# EUROPEAN PATENT APPLICATION

(11) **EP 1 878 388 A1**
(43) Date of publication of application: **16.01.2008**
(21) Application number: 07013476.2
(22) Date of filing: 10.07.2007
(51) Int. Cl.: A61B 8/00

(54) **Ultrasonic probe with a device for removing bubbles**

(30) Priority: 12.07.2006 KR 20060065288
(71) Applicant: MEDISON CO., LTD., Kangwon-do 250-870 (KR)
(72) Inventor: Cho, Joo Yeon, Seoul 135-280 (KR)
(74) Representative: Lorenz, Markus

(57) **Abstract**

Embodiments of the present invention may provide an ultrasonic probe, which is configured to drive an ultrasonic element and to remove bubbles remaining in liquid filled for propagation of ultrasonic waves. The ultrasonic probe (100, 200) of the present invention has a housing (112), a cover (113) and a device for removing bubbles (130, 130', 230). The housing (112) supports an ultrasonic element (111a) so that it can be reciprocated therein. The cover (113) is joined to the housing (112) to define an enclosed space (115), in which the ultrasonic element (111a) is reciprocated. The bubble-removing device (130, 130', 230) has a tube (131, 231), which is joined at one end to a portion of the housing (112) opposed to the cover (113) and is closed at the other end (134, 234). A communication passage (132a, 132a', 232a) is formed at the one end of the tube. The communication passage (132a, 132a', 232a) has a shape of a funnel or a truncated cone, which becomes narrow as it goes toward the other end (134, 234) of the tube. The bubble-removing device (130, 130', 230) removes the bubbles (116a) from the liquid (116) filled in the enclosed space (115) for propagation of ultrasonic waves and then accumulates the removed bubbles.

## Description

The present application claims priority from Korean Patent Application No. 10-2006-0065288 filed on July 12, 2006, the entire subject matter of which is incorporated herein by reference.

### BACKGROUND

### 1. Field

The present invention generally relates to ultrasonic probes, and more particularly to an ultrasonic probe configured to reciprocate an ultrasonic element within a certain range and remove bubbles from liquid provided for propagating ultrasonic waves.

### 2. Background

An ultrasonic diagnostic apparatus is widely used to diagnose a subject by visualizing a portion within the subject's body. The ultrasonic diagnostic apparatus has an ultrasonic probe, which is used to make contact with the skin of the subject.

The ultrasonic probe has an ultrasonic element therein. The ultrasonic element serves to generate ultrasonic waves and transmit/receive ultrasonic waves. The ultrasonic probe can be classified as a linear, convex or circular type according to an array formation of ultrasonic vibrating elements constructing the ultrasonic element. Further, the ultrasonic probe can be classified as a two-dimensional image probe or a three-dimensional image probe according to the type of obtained images. Recently, an ultrasonic probe, which is configured to reciprocate the ultrasonic element within a certain range, has been used to obtain a more accurate two-dimensional or three-dimensional image.

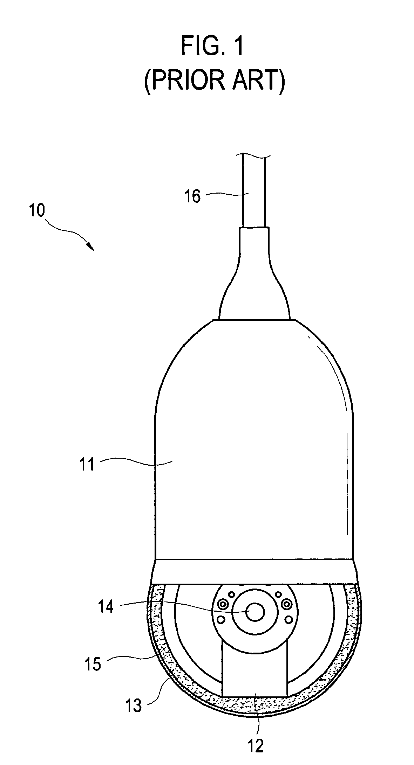

Fig. 1 is a partially cutaway front view of a prior art ultrasonic probe for use in acquiring a three-dimensional image. The ultrasonic probe 10 includes a case 11, which is sized and configured to be grasped by an operator or user and accommodates various parts therein. An ultrasonic element 12 is disposed adjacent to a front end of the case 11. The ultrasonic element 12 is connected to a main body of an ultrasonic diagnostic apparatus (not shown) via a cable 16. A drive device (not shown), which causes the ultrasonic element 12 to be reciprocated around a shaft 14 within a certain range, is provided inside the case 11. A cover 13, which covers and protects the ultrasonic element 12 and may be in contact with the skin of a subject, is joined to the front end of the case 11. An enclosed space defined by the cover 13 and the case 11, wherein the ultrasonic element 12 is reciprocated, is filled with oil 15 in order to facilitate the propagation of ultrasonic waves.

Oil 15 is injected into the enclosed space after said space is established in the ultrasonic probe 10. The oil 15 is injected to replace the air in the enclosed space. During assembly of the ultrasonic probe 10, injecting the oil and discharging the air are carried out simultaneously. However, in the enclosed space, there are parts such as ultrasonic element 12, bearings and screw threads of the drive device, etc. Such parts have a number of concave-convex portions thereon. When the oil enters such concave-convex portions, the air in the concave-convex portions is not discharged smoothly, thereby remaining as bubbles in the injected oil 15. Therefore, the bubbles remaining in the oil float in the oil after the assembly of the ultrasonic probe 10 is ended.

Thus, there is a problem with a prior art ultrasonic probe in that the ultrasonic waves are not allowed to freely propagate due to the bubbles remaining in the oil. This clearly makes it difficult to obtain accurate images.

### BRIEF DESCRIPTION OF THE DRAWINGS

Arrangements and embodiments may be described in detail with reference to the following drawings in which like reference numerals refer to like elements and wherein:

Fig. 1 is a partially cutaway front view of a prior art ultrasonic probe for use in acquiring a three-dimensional image;

Fig. 2 is a partially cutaway perspective view of an ultrasonic probe constructed in accordance with a first embodiment of the present invention;

Fig. 3 is a sectional view showing a device for removing bubbles;

Fig. 4 is a partial sectional view of the ultrasonic probe shown in Fig. 2 when the bubbles are removed;

Fig. 5 is a view showing a state when the accumulated bubbles are not discharged again;

Fig. 6 is a sectional view showing an alternative to the bubble-removing device shown in Fig. 3;

Fig. 7 is a sectional view of the bubble-removing device shown in Fig. 6 when the bubbles are not discharged again;

Fig. 8 is a partially cutaway perspective view of an ultrasonic probe constructed in accordance with a second embodiment of the present invention;

Fig. 9 is a sectional view of a device for removing bubbles shown in Fig. 8; and

Fig. 10 is a bottom view of a connector of the bubble-removing device shown in Fig. 9.

### DETAILED DESCRIPTION OF THE PRESENT INVENTION

A detailed description may be provided with reference to the accompanying drawings. The terms "frontward" and "rearward" are intended to mean a direction in which an ultrasonic probe is brought into contact with the skin of a subject and an opposite direction thereof, respectively. One of ordinary skill in the art may realize that the following description is illustrative only and is not in any way limiting. Other embodiments of the present invention may readily suggest themselves to such skilled persons having the benefit of this disclosure.

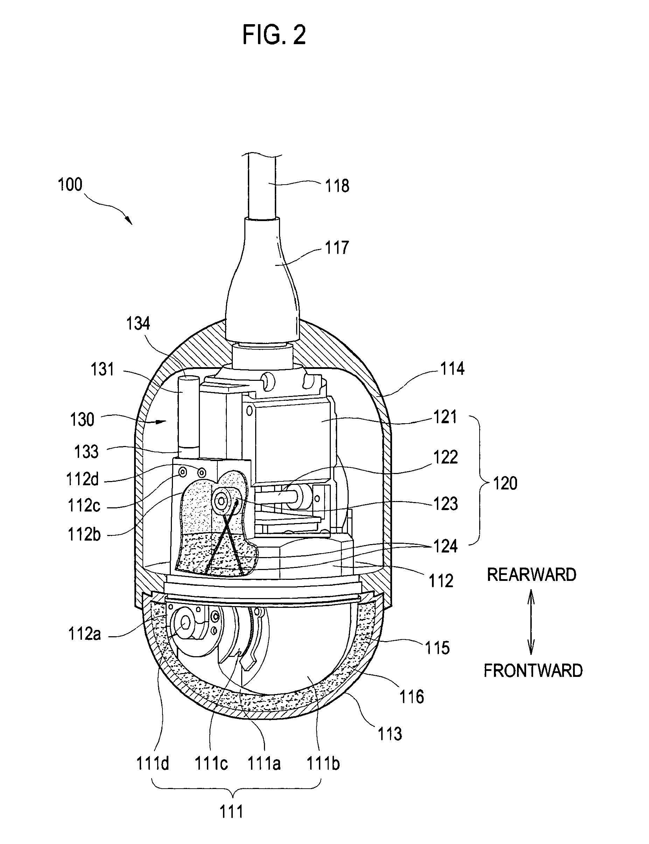

Fig. 2 is a partially cutaway perspective view of an ultrasonic probe, which is constructed in accordance with a first embodiment of the present invention.

The ultrasonic probe 100 comprises the following: an ultrasonic element assembly 111; a housing 112 for supporting the ultrasonic element assembly 111 so that the ultrasonic element assembly 111 is reciprocated therein; a cover 113 for covering and protecting the ultrasonic element assembly 111; a case 114 configured to be hand-held by a user and secure the housing 112 and the cover 112 thereto; a drive device 120 mounted to the housing 112 for reciprocating the ultrasonic element assembly 111 within a certain range; and a device for removing bubbles 130. Various electrical signals of the ultrasonic probe 100 are transmitted and received to and from a main body of an ultrasonic diagnostic apparatus (not shown) using a cable 118. The cable 118 is extended from a rearward portion of the case 114 through a flexible bushing 117.

The ultrasonic element assembly 111 includes: an ultrasonic element 111a; an element holder 111b for fixing and supporting the ultrasonic element 111a; a rope holder 111c for attaching a wire rope 124 thereto; and a shaft 111d serving as a pivot, around which the ultrasonic element assembly 111 is pivotally reciprocated.

The ultrasonic element 111a comprises a number of ultrasonic vibrating elements including a piezoelectric material, a backing layer and a matching layer. The ultrasonic element 111 a of this embodiment may be formed so as to allow a number of ultrasonic vibrating elements to be curvilinearly arrayed. The ultrasonic element 111 a is connected to the main body of the ultrasonic diagnostic apparatus using the cable 118. The ultrasonic element generates ultrasonic waves from the signals, which are transmitted from the main body of the ultrasonic diagnostic apparatus, and transmits the same. The ultrasonic element may further be configured to receive the reflected ultrasonic waves, convert the received ultrasonic waves into electrical signals and output the electrical signals to the main body of the ultrasonic diagnostic apparatus.

The element holder 111b supports the ultrasonic element 111a at both sides thereof. The shaft 111d is formed at both sides of the element holder 111b. The rope holder 111c, which is coaxially positioned together with the shaft 111d, is secured to the element holder 111b. The shaft 111d is rotatably joined to a shaft-supporting portion 112a formed at a frontward end of the housing 112.

The housing 112 is opened at its frontward side so that the ultrasonic element assembly 111 is placed therein. The housing 112 is closed at its rearward side. A portion of the drive device 120 is introduced inside the housing 112 through an extended portion 112b formed at the rearward side of the housing 112.

The drive device 120 includes the following: an electrical motor 121; a connecting shaft 122, which is connected to a rotating shaft of the electrical motor 121 at its one end and is introduced inside the housing 112 through the extended portion 112b at its other end; a rope-driving pulley 123 coupled to the other end of the connecting shaft 112; and the wire rope 124 hung between the rope-driving pulley 123 and the rope holder 111c.

The electrical motor 121 receives electrical power required for its operation from the main body of the ultrasonic diagnostic apparatus using the cable 118 while being controlled by the main body of the ultrasonic diagnostic apparatus. The normal or reverse rotation of the electrical motor 121 effectuates clockwise or counterclockwise rotational movement of the ultrasonic element assembly 111 around the shaft 111d through the connecting shaft 122, the rope-driving pulley 123, the wire rope 124 and the rope holder 111c. Since the electrical motor 121 effectuates the normal or reverse rotation, the ultrasonic element assembly 111 can be reciprocated accordingly within a certain range.

As illustrated in Fig. 1, only a part of the drive device 120 (e.g., rope-driving pulley 123 and wire rope 124) is disposed inside the housing 112. However, the housing 112 may be configured to accommodate the entire drive device 120. Further, the drive device 120 shown in Fig. 1 reciprocates the ultrasonic element assembly 111 by using the wire rope. However, spur gears, worm gears, racks-pinions, cams or the like may be employed as a driving mechanism of the drive device 120.

The pivotally reciprocable ultrasonic element assembly 111 cannot be brought into direct contact with the skin of the subject. Thus, the cover 113 is joined to the frontward edge of the housing 112 for contacting the skin of the subject. The cover 113 covers and protects the ultrasonic element assembly 111 while defining an enclosed space 115, wherein the ultrasonic element assembly 111 is reciprocated, together with the housing 112.

The cover 113 may be formed from a high molecular material similar to a human body so as to possess hardness or elasticity, which is deformable according to a reaction force of the skin of the subject. In Fig. 1, the cover 113 is dome-shaped. When the ultrasonic probe is configured such that a linear array ultrasonic element is reciprocated (unlike this embodiment), the cover 113 may be formed to have a flat shape.

The case 114, which is cylinder-shaped, is joined to the frontward edge of the housing 112 at its opened one end. While the cover 113 is joined to the housing 112 and the case 114 fixes and supports the housing 113 as shown in Fig. 1, the housing 112 may be joined to the case 114 and the cover 113 may be joined to the case 114. Alternatively, the housing 112 may be omitted and the case 114 may secure the drive device 120 and support the ultrasonic element assembly 111 so that it can be reciprocated.

Where the enclosed space 115 is filled with air, ultrasonic waves, which are transmitted from and received by the ultrasonic element 111a, are difficult to propagate through air. For this reason, a liquid medium 116 is filled in the enclosed space 115 to facilitate the unobstructed propagation of ultrasonic waves. Oil or saline solution can be used as the liquid medium 116.

The liquid medium 116 is injected into the housing 112 after the ultrasonic element assembly 111 and the cover 113 are assembled into the housing 112. The extended portion 112b of the housing 112 is formed with an injection port 112c for injecting the liquid medium and an exhaust port 112d, through which the air replaced by injection of the liquid medium is exhausted. If the injection of the liquid medium is ended, then the injection port 112c and the exhaust port 112d are sealed up. This accomplishes the enclosed space 115, wherein the ultrasonic element assembly 111 is reciprocated.

The surfaces of the components located in the enclosed space 115 (e.g., a surface of the ultrasonic element assembly 112, an inner surface of the housing 112, surfaces of the drive device 120, etc.) are not even. Rather, they have various concave-convex portions. Therefore, when the liquid medium is injected, the air in said concave-convex portions remains in the liquid medium to thereby form bubbles. Such bubbles obstruct the propagation of the ultrasonic waves to thereby deteriorate the performance of the ultrasonic probe 100. However, the bubble-removing device 130 removes the bubbles from the liquid medium and accumulates the removed bubbles.

The bubble-removing device 130 is joined to a rearmost portion (e.g., extended portion 112b) among portions of the housing 112, which are opposed to the cover 113. The bubble-removing device 130 comprises a tubular member and a communication portion for allowing the bubbles to pass into the tubular member and restricting a counter passing thereof. The tubular member includes a straight tube 131, which is made from plastic or rubber. The tube 131 is joined to the extended portion 112b at its one end in such a manner that it penetrates a wall of the extended portion 112b. The tube 131 is closed at its other end 134. The one end of the tube 131 consists of a connector 133. The tube 131 is located in the case 114 so that the other end 134 of the tube 131 is farther away from the cover 113 than the connector 133. The communication portion is disposed in the connector 133.

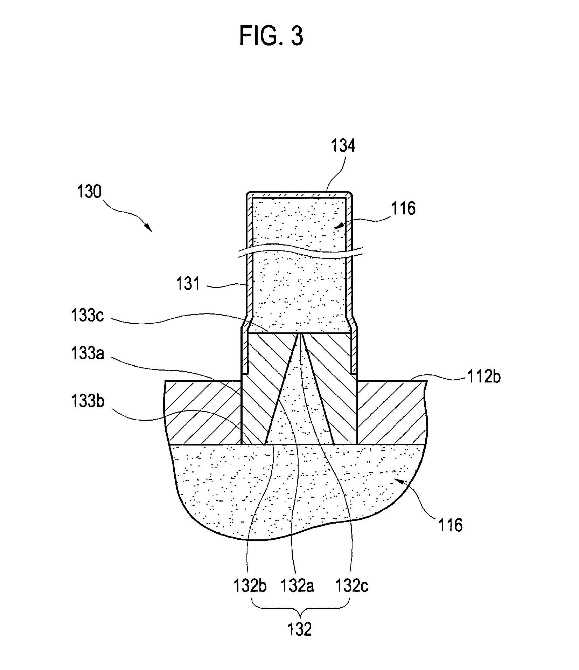

Fig. 3 is a sectional view of the bubble-removing device 130.

The connector 133 includes the following: a cylindrical connector body 133a; one end 133b facing the inside of the housing 112; and the other end 133c configured to join a body of the tube 131. The one end 133b of the connector is formed at its middle portion with an opening. The connector body 133a is liquid-tightly joined to the rearmost portion of the housing (e.g., extended portion 112b) whereby the bubble-removing device 130 is fixed to the rearmost portion of the housing.

The communication portion 132, which is disposed at the one end of the tube 131 (more specifically, the connector 133), includes the following: a communication passage 132a having a shape of a truncated cone; and an inlet 132b and an outlet 132c formed at both ends of the communication passage 132. The communication portion 132 is located in the connector body 133a such that the outlet 132c faces the inside of the tube 131 while the inlet 132b faces the inside of the housing 112. The inlet 132b is coupled to the opening formed at the one end 133b of the connector. The communication passage 132a becomes narrow as it goes toward the outlet 132c. Therefore, the size of the outlet 132c is smaller than that of the inlet 132b. The size of the outlet 132c is determined such that one of the bubbles remaining in the liquid medium can pass through the outlet 132c.

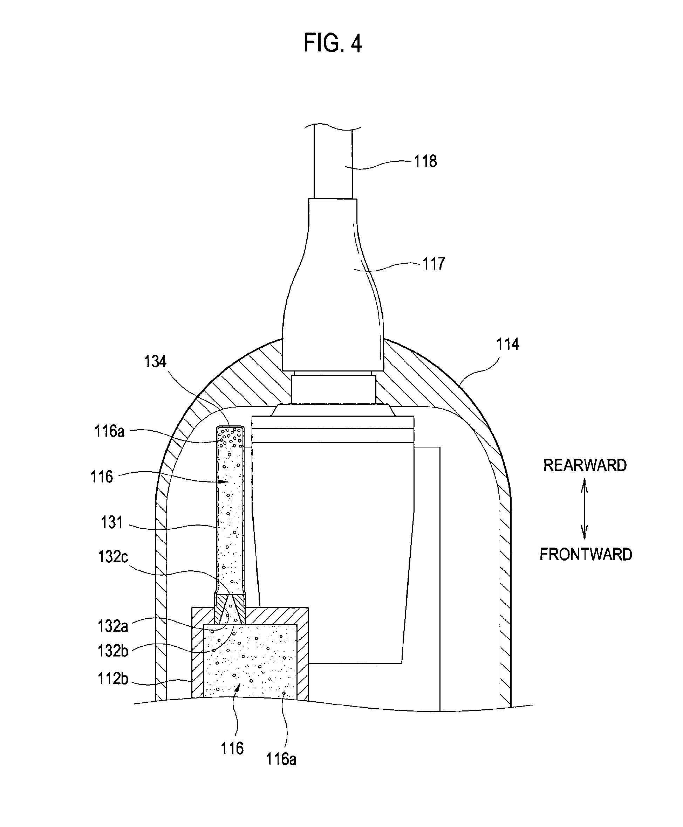

Fig. 4 is a partially sectional view of the ultrasonic probe 100, which shows that the bubbles are being accumulated within the tube 131. Fig. 4 illustrates a state where the ultrasonic probe 100 is placed vertically so that its cover side faces the ground (hereinafter, such a state is referred to as "a bubble-removing state").

Referring to Fig. 4, in the bubble-removing state, the bubbles 116a float up rearward (toward the side opposite to the cover 113) due to their buoyancies. The floating up bubbles 116a reach the vicinity of the inlet 132b of the communication portion. Then, the bubbles 116a enter the inlet 132b and gather in the communication passage 132a. When only a single bubble enters the communication passage 132, said bubble 116a moves up along a sloped surface of the communication passage 132a due to its buoyancy and passes through the outlet 132c to enter the inside of the tube 131. When a number of bubbles enters the communication passage 132a, said bubbles 116a gather in the vicinity of the outlet 132c and then pass through the outlet 132c in order to enter the inside of the tube 131 due to their buoyancies one by one.

When the ultrasonic probe 110 is left in the bubble-removing state, the bubbles 116a continuously enter the inside of the tube 131 and can be accumulated inside the tube 131 from the vicinity of the other end 134 of the tube 131. The ultrasonic probe 100 may be left in the bubble-removing state when the ultrasonic probe 100 is placed for a certain period of time after ending assembly in the state where its cover side faces the ground. Alternatively, the ultrasonic probe 100 may be left in the bubble-removing state when ultrasonic diagnosis can be carried out using the ultrasonic probe 100. In both cases, removing and accumulating the bubbles take place as described above. Particularly, when the cover 113 is made from an elastic material, if the cover is slightly pressed toward the inside of the ultrasonic probe during the ultrasonic diagnosis, then the bubbles 116a, which gather in the communication passage 132a, are allowed to enter the inside of the tube 131 more easily due to the pressure applied to the liquid medium.

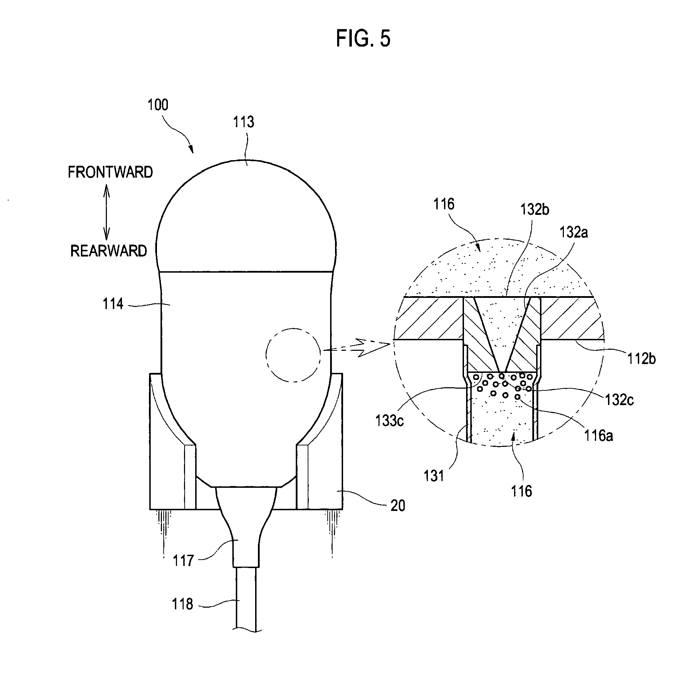

Fig. 5 shows a state where the accumulated bubbles are not discharged again (contrary to the state shown in Fig. 4). When the ultrasonic probe 100 is not utilized, the ultrasonic probe 100 may be placed on any fixture (e.g., a probe holder 20 provided on the main body of the ultrasonic diagnostic apparatus). In such a case, the bubbles 116a accumulated within the tube 131 float up along the tube 131 toward the outlet 132c. However, since the diameter of the outlet 132c is very narrow, the floating up bubbles 116a are difficult to move through the outlet 132c toward the inlet 132b. Furthermore, the floating up bubbles 116a are restricted from being discharged to the inside of the housing through the rearmost portion of the housing by the other end 133c of the connector. Although a very small amount of bubbles, among the accumulated bubbles, can enter the inside of the housing 112 through the outlet 132c, such bubbles can be removed from the liquid medium 116 again whenever the ultrasonic probe 100 is in the bubble-removing state. Consequently, the bubbles 116a remaining in the liquid medium 116 are allowed to be accumulated in the tube 131 while being removed successively. Even when the ultrasonic probe 100 is placed horizontally, the bubbles already accumulated in the tube 131 are difficult to be discharged to the housing 112 due to the structure of the bubble-removing device 130.

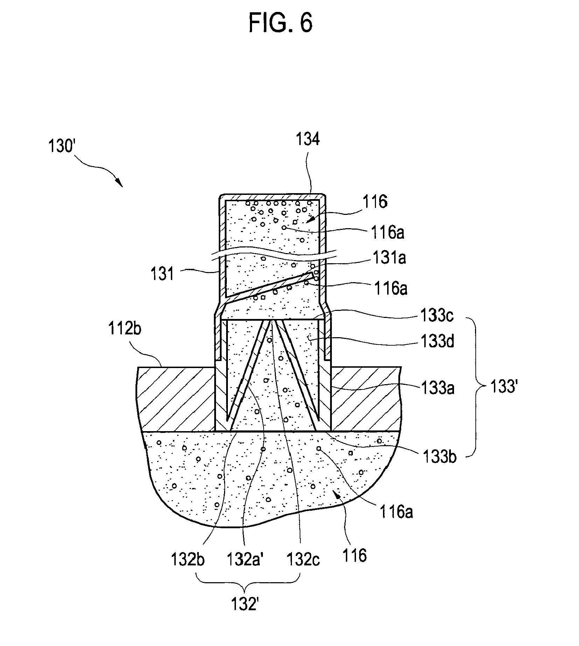

Fig. 6 shows an alternative to the bubble-removing device shown in Fig. 3 in the bubble-removing state. In a bubble-removing device 130' shown in Fig. 6, the tube 131 includes a blocking plate 131a therein. The blocking plate 131a is sloped toward the other end 134 and blocks the inside of the tube 131 with a gap, which is sized such that the bubbles can move therethrough. A plurality of blocking plates may be included inside the housing 112. The communication portion 132' includes a funnel-shaped or a truncated cone-shaped communication passage 132a', which becomes narrow as it proceeds toward the other end 134. The connector 133' has a barrel shape to define a cavity 133d together with the communication portion 132'. In the bubble-removing state, the bubbles 116a move through the outlet 132c and enter the inside of the tube 131. Also, the bubbles move up along the blocking plate 131 a and reach the other end 134 of the tube 131 so as to be accumulated in the tube 131.

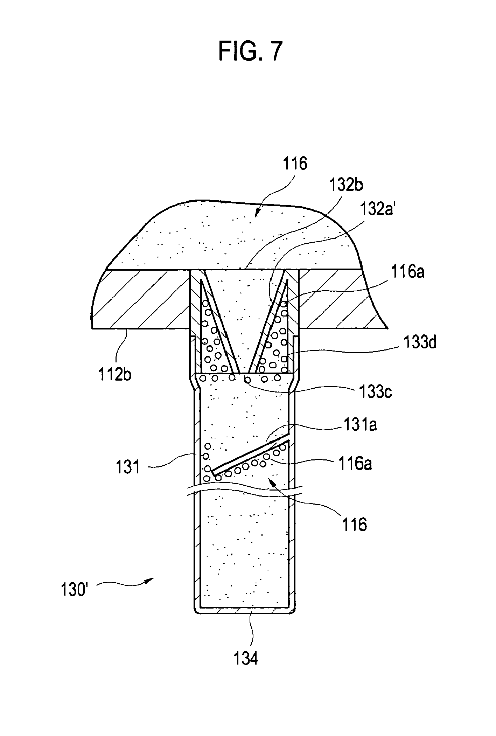

Fig. 7 is a sectional view of the bubble-removing device 130' when the ultrasonic probe is placed in an opposite state to the bubble-removing state.

In the state shown in Fig. 7, the bubbles 116a accumulated in the tube 131 float up toward the outlet 132c along the tube 131 once again. In such a case, a substantial portion of the accumulated bubbles 116a are caught between the inner wall of the tube 131 and the blocking plate 131a and are retained therein. The uncaught bubbles 116a go through the gap between the inner wall of the tube 131 and the blocking plate 131a and then float up toward the outlet 132c. However, said bubbles cannot easily pass through the outlet 132c toward the inlet 132b due to the very narrow outlet 132c. Further, since the cavity 133d has a broader cross-sectional area than that of the outlet 132c, most of the uncaught and floating up bubbles enter the cavity 133d.

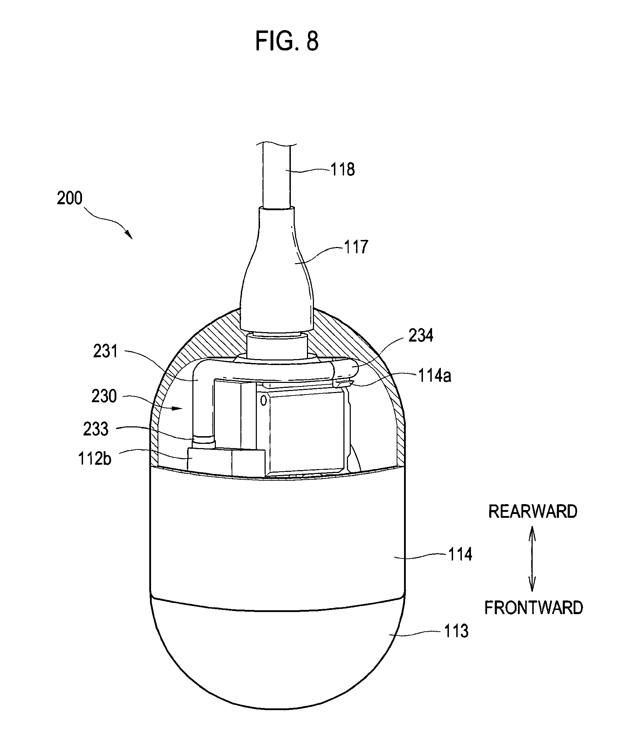

Fig. 8 is a partially cutaway perspective view of an ultrasonic probe, which is constructed in accordance with a second embodiment of the present invention. The ultrasonic probe 200 of this embodiment has the same configuration as the ultrasonic probe 100 of the first embodiment, except that the bubble-removing device is configured to allow the injection of the liquid medium.

Referring to Fig. 8, the bubble-removing device 230 comprises a tubular member and a communication portion (not shown) for permitting the bubbles to pass into the tubular member and restricting a counter passing thereof. The tubular member is joined to the rearmost portion (e.g., extended portion 112b) of the housing at its one end in such a manner that it penetrates the wall of the extended portion 112b. The other end of the tubular member is closed and configured to allow the injection of the liquid medium. The communication portion is disposed at the one end of the tubular member.

The tubular member includes a tube 231, which is made from a soft material (e.g., rubber) or a hard material (e.g., plastic) and is bent at one portion thereof. The tube 231 extends rearward vertically from the rearmost portion 112b of the housing 112 and is bent in the vicinity of the rearward inner wall of the case 114. It further extends along the rearward inner wall of the case 114. In the vertical extending portion of the tube 231, there may be provided the blocking plate 131a shown in Fig. 6.

One end of the tube 231 consists of a connector 233, which is joined to a medium injection port of the rearmost portion of the housing. The closed other end 234 of the tube 231 consists of an injection port 234a fitted to the tube 231 and a plug 234b for sealing the injection port 234a. The injection port 234a is retained in the inner wall of the case 114 by being engaged to a hook-shaped injection port holder 114a provided on a rearmost portion of the inner wall of the case 114, thereby being located farther away from the cover 112 than the connector 233.

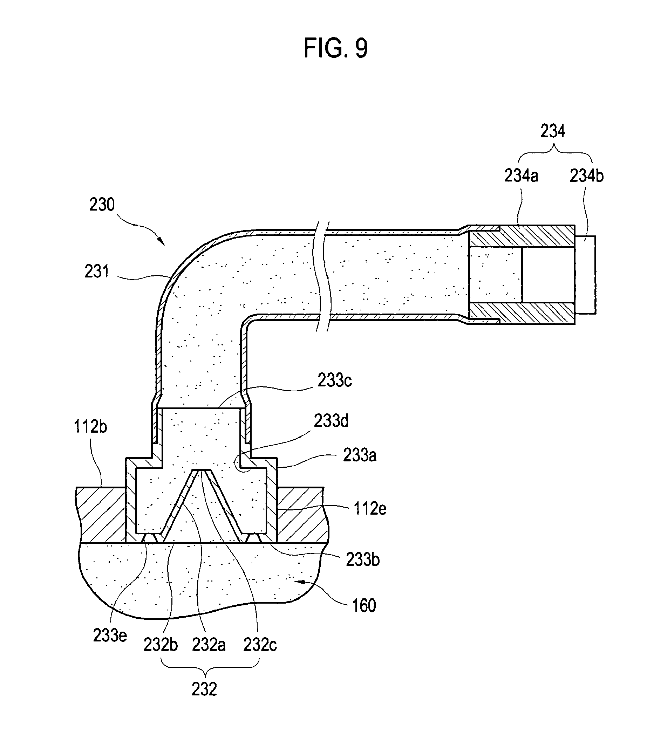

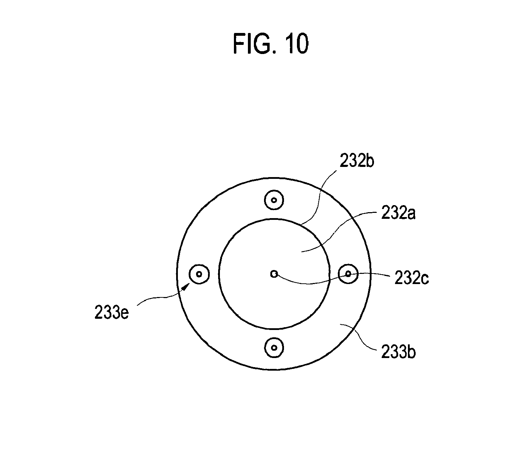

Fig. 9 is a sectional view of the bubble-removing device 230 shown in Fig. 8. Fig. 10 is a bottom view of the connector 233 of the bubble-removing device 230.

The connector 233 includes the following: a barrel-shaped connector body 233a; one end 233b facing the inside of the housing 112; and the other end 233c to which a body of the tube 231 is coupled. The one end 233b is formed with an opening.

The communication portion 232, which is disposed at the one end of the tube 231 (more specifically, the connector 233), includes the following: a funnel-shaped communication passage 232a, which becomes narrow as it proceeds toward the other end 234; and an inlet 232b and an outlet 232c formed at both ends of the communication passage 232a. The inlet 232b is coupled to the opening formed at the one end 233b of the connector. The outlet 232c is opened toward the inside of the tube 231. The size of the outlet 232c is much smaller than that of the inlet 232b due to the funnel shape of the communication passage 232a. The size of the outlet 232c is determined such that one of the bubbles remaining in the liquid medium can pass through the outlet 232c. A cavity 233d is formed between the inner wall of the connector body 233a and the communication portion 232 in the connector 233.

The communication portion 233 further includes a plurality of funnel-shaped through-holes 233e at its one end 233b. The through-holes 233e are located adjacent to the inlet 232c. The through-hole 233e becomes narrow as it proceeds toward the inside of the connector body 233a.

If the liquid medium is filled in the enclosed space to some level during assembly of the ultrasonic probe 200, then the bubble-removing device 230 is joined to the medium injecting port 112e and the injection of the liquid medium is continued. In such a case, the liquid medium is injected into the housing 112 through the outlet 232c of the communication portion and the through-holes 233e. At the same time, the air inside the housing 112 can be discharged through one of the outlets 232c and the through-holes 233e. If the liquid medium is injected into the vicinity of the injection port 234a, then the injection port 234a is plugged with the plug 234b and the injection port 234a is secured to the case 114. Thereafter, the assembly of the ultrasonic probe 200 is continued. As such, the bubble-removing device 200 may be used to inject the liquid medium during the assembly of the ultrasonic prove 200 and remove the bubbles remaining in the filled liquid medium after the ultrasonic probe 200 is completely assembled.

Embodiments of the present invention may provide an ultrasonic probe. The ultrasonic probe of the present invention can simply and successively remove the bubbles remaining in the liquid medium filled in the enclosed space, wherein the ultrasonic element is reciprocated. When the ultrasonic probe is vertically placed so that the cover faces the ground or when the ultrasonic diagnosis is carried out with the ultrasonic probe, the bubbles can be removed from the liquid medium.

An ultrasonic probe may be provided. The ultrasonic probe of the present invention may comprise an ultrasonic element, a housing, a drive device, a cover, a liquid medium and a device for removing bubbles. The housing may support the ultrasonic element so that the ultrasonic probe can be reciprocated therein. The drive device may be secured to the housing to reciprocate the ultrasonic element. The cover may be joined to the housing to define an enclosed space in which the ultrasonic element is reciprocated. The liquid medium may be filled in the enclosed space. The bubble-removing device may include a tubular member and a communication passage. The tubular member may be joined at one end to a portion of the housing opposed to the cover to be in communication with the enclosed space. The tubular member may be located so that a closed other end is farther away from the cover than the one end. The communication passage may be disposed at the one end and becomes narrow as it proceeds toward the other end. The communication passage may have a shape of a funnel or a truncated cone. A blocking plate may be provided inside the tubular member. The blocking plate may partially close the inside of the tubular member and be sloped toward the other end.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasonic probe (100, 200), comprising:
an ultrasonic element (111a);
a housing (112) for reciprocably supporting the ultrasonic element (111a);
a drive device (120) secured to the housing (112) for reciprocating the ultrasonic element (111a);
a cover (113) joined to the housing (112) to define an enclosed space (115), the ultrasonic element (111a) being reciprocated in the enclosed space (115);
a liquid medium (116) filled in the enclosed space (115); and
a device for removing bubbles (130, 130', 230) including: a tubular member (131, 231) joined at one end to a portion of the housing (112) opposed to the cover (113) to be in communication with the enclosed space (115) and being situated so that a closed other end (134, 234) is farther away from the cover (113) than the one end; and a communication passage (132a, 132a', 232a) disposed at the one end and being narrow as going toward the other end (134, 234).

2. The ultrasonic probe of Claim 1, wherein the communication passage (132a, 132a', 232a) has a shape of a funnel or a truncated cone.

3. The ultrasonic probe of Claim 1, wherein a blocking plate (131a) is provided inside the tubular member (131, 231) and the blocking plate partially closes the inside of the tubular member and is sloped toward the other end (134, 234).
